(19) Europäisches Patentamt
European Patent Office
Office européen des brevets

(11) Publication number: 0 019 308
B1

(12) # EUROPEAN PATENT SPECIFICATION

(45) Date of publication of patent specification: 16.06.82

(51) Int. Cl.³: C 07 D 257/04

(21) Application number: 80200260.0

(22) Date of filing: 23.06.78

(60) Publication number of the earlier application in accordance with Art. 76 EPC: 0000272

(54) **Tetrazole derivatives and a process for their preparation.**

(30) Priority: 24.06.77 US 809585

(43) Date of publication of application: 26.11.80 Bulletin 80/24

(45) Publication of the grant of the patent: 16.06.82 Bulletin 82/24

(84) Designated Contracting States: BE CH DE FR GB LU NL SE

(56) References cited:
GB - A - 567 353
US - A - 4 057 631

(73) Proprietor: SMITHKLINE CORPORATION
1500 Spring Garden Street P.O. Box 7929
Philadelphia Pennsylvania 19101 (US)

(72) Inventor: Berges, David Alan
18 Buckwalter Road
Phoenixville, Pennsylvania 19460 (US)

(74) Representative: Waters, David Martin, Dr. et al,
P.O. Box 39
Welwyn Garden City, Hertfordshire AL7 1EY (GB)

EP 0 019 308 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Tetrazole derivatives and a process for their preparation

This invention relates to tetrazole derivatives and a process for their preparation.

British Specification No. 567,335 discloses a process for preparing substituted tetrazoles of formula:

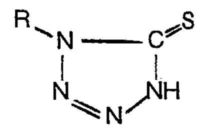

where R is a substituted or unsubstituted alkyl group. However no utility is disclosed for these compounds.

The compounds of this invention have a different structure from these known compounds because one nitrogen atom adjacent to the thione group has an alkyleneamino-alkylene carboxylic acid substituent in place of the substituted or unsubstituted alkyl group.

According to the invention there is provided a compound of formula (I):—

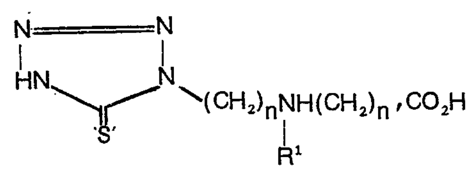

(I)

its alkali metal and ammonium salts; where n is from two to four inclusive, n′ is from one to four inclusive and $R^1$ is hydrogen or lower alkyl.

The tetrazole derivatives of this invention are useful for preparing cephalosporin derivatives disclosed and claimed in our European Patent Application No. 7830076.3. (Publication Number 0 000 272).

Compounds of formula (I) exist in tautomeric forms:—

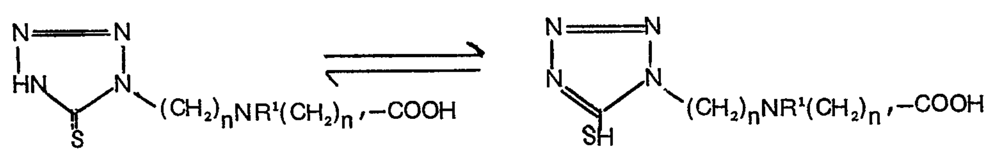

and it is understood that reference to formula (I) include reference to all such tautomers.

Herein lower alkyl means such a group containing up to four carbon atoms.

Preferably n is 2.

Preferably n′ is 1.

Where $R^1$ is lower alkyl it is preferably methyl but $R^1$ is preferably hydrogen.

The compounds of this invention can be made by reacting an aminoalkyltetrazolyl thione derivative of formula (II):—

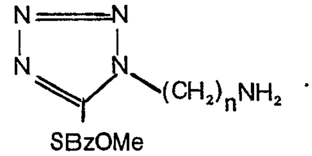

(II)

where n is as defined with reference to formula (I) and BzOMe is p-methoxybenzyl with a bromoester of formula (III):—

$$Br(CH_2)_{n'}CO_2C_2H_5$$

(III)

and where R[1] is lower alkyl optionally reacting the ester of the compound of formula (I) so obtained with an alkylating agent which introduces a lower alkyl group thereafter removing the p-methoxy benzyl group, hydrolysing the ester group and optionally converting the free acid into the salt.

The following Examples illustrate the invention.

Example 1

A mixture of 21.5 g. (11.4 mmol) of 1 - [2 - (acetylamino)ethyl] - 1,4 - dihydro - 5*H* - tetrazole - 5 - thione in 300 ml. of 6N hydrochloric acid was heated at reflux for 3.5 hours. The mixture was filtered after cooling to room temperature. The filtrate was concentrated to small volume. The residual liquid was diluted with i-propanol. The solid which precipitated was filtered, washed and dried *in vacuo* to give 13.7 g. of 1 - (2 - aminoethyl) - 1,4 - dihydro - 5*H* - tetrazole - 5 - thione, hydrochloride (66.1% yield) mp 232—233.5°C.

To a solution of 22.8 g. (12.5 mmol) of 1 - (2 - aminoethyl - 1,4 - dihydro - 5*H* - tetrazole - 5 - thione, hydrochloride in 100 ml. of N,N-dimethylformamide and 100 ml. of acetone was added 34.3 ml. (25 mmol) of triethylamine. To the resulting suspension was added slowly a solution of 19.5 g. (12.5 mmol) of *p*-methoxybenzyl chloride in 30 ml. of acetone. After stirring at room temperature for 15 hours, the mixture was filtered. The filtrate was evaporated to dryness. The residue was taken up in 350 ml. of 5% $NaHCO_3$, and extracted with ethyl acetate. The combined extract was dried ($MgSO_4$) and evaporated to dryness to give an oil which was chromatographed on a silica gel column, eluting with a gradient of 5—10% ethanol in chloroform. Fractions containing product by thin layer chromatography were pooled, and evaporated to dryness to give 1 - (2 - aminoethyl) - 5 - (4 - methoxybenzylthio) - 1*H* - tetrazole as a brown oil (26 g., 80%). An analytical sample of the crystalline amine hydrochloride (mp 148—150°) was obtained by treating the product with an ethereal HCl solution.

To a solution of 15.0 g. (56 mmol) of 1 - (2 - aminoethyl) - 5 - [(4 - methoxybenzyl)thio] - 1*H* - tetrazole in 70 ml. of dry tetrahydrofuran was added 7.7 ml. (56 mmol) of triethyl-amine, and 6.2 ml. (56 mmol) of ethyl bromoacetate. After stirring at room temperature for 15 hours, the mixture was filtered, and the filtrate was evaporated *in vacuo* to dryness. The residue was dissolved in 70 ml. of chloroform and de-colorized with charcoal. The filtrate was chromatographed on silica gel, eluting with a gradient of 0—15% ethyl acetate in chloroform. Fractions containing product by thin layer chromatography were pooled and evaporated to dryness to give 12.5 g. (62% yield) of 1 - [2 - [[(carbethoxy)methyl]amino] - ethyl] - 5 - [(4 - methoxybenzyl)thio] - 1*H* - tetrazole as a brown oil.

To a solution of 12.5 g. (35.6 mmol) 1 - [2 - [[(carbethoxy)methyl]amino]ethyl] - 5 - [(4 - methoxybenzyl)thio] - 1*H* - tetrazole in 250 ml. of methanol and 65 ml. of water was added a solution of 25.5 g. (80 mmol) of mercuric acetate in 80 ml. of water. The mixture was stirred at room temperature for 15 hours and at reflux for 1 hour. After thorough cooling, the mixture was treated with hydrogen sulfide gas for 1.5 hours. The dark mixture was heated over a steam bath for 1.5 hours and filtered. The filtrate was evaporated *in vacuo* to dryness. The residue was recrystallized from ethyl acetate to give 5.9 g. of 1 - [2 - [(carboxymethyl)amino]ethyl] - 1,4 - dihydro - 5*H* - tetrazole - 5 - thione (82.8% yield) mp 215—220° dec.

Example 2

To a solution of 10 mmol of 1 - [2 - [[(carbethoxy) - methyl]amino]ethyl] - 5 - [4 - methoxybenzyl)thio] - 1*H* - tetrazole and 10 mmol of triethylamine in 20 ml. of dry tetrahydrofuran is added 10 mmol of methyliodide. After stirring at room temperature for 24 hours, the mixture is filtered. The filtrate is stripped *in vacuo* to dryness, and the residue is dissolved in chloroform and chromatographed on silica gel eluting with a gradient of ethylacetate in chloroform. Evaporation of the product-containing fractions gives 1 - [2 - [[(carbethoxy)methyl]methylamino]ethyl] - 5 - [(4 - methoxl - benzyl)thio] - 1*H* - tetrazole. Deblocking with mercuric acetate as above gives 1 - [2 - [(carboxymethyl)methylamino]ethyl] - 1,4 - dihydro - 5*H* - tetrazole - 5 - thione.

**Claims**

1. A compound of formula (I):

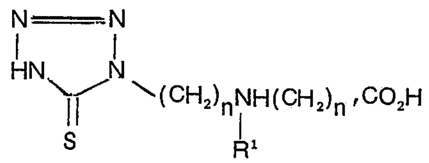

(I)

is alkali metal and ammonium salts, where n is from two to four inclusive, and n' is from one to four inclusive and $R^1$ is hydrogen or $C_1$—$C_4$ alkyl.

2. A compound as claimed in claim 1 where $R^1$ is hydrogen.

3. A compound as claimed in claim 1 where $R^1$ is methyl.

4. A compound as claimed in any one of claims 1 to 3 where n is 2 and n' is 1.

5. A process for preparing a compound as claimed in claim 1 which comprises reacting an aminoalkyltetrazolyl thione derivative of formula (II):—

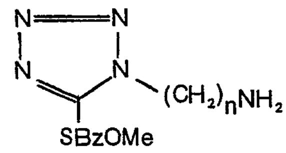

(II)

where n is as defined in claim 1 and BzOMe is p-methoxybenzyl with a bromoester of formula (III):—

$$Br(CH_2)_{n'}CO_2C_2H_5$$

(III)

optionally thereafter reacting the ester of the compound of formula (I) so produced with an alkylating agent which introduces a lower alkyl group, thereafter removing the p-methoxybenzyl group, hydrolysing the ester group and optionally converting the free acid to a salt.

**Revendications**

1. Composé de formule (I):

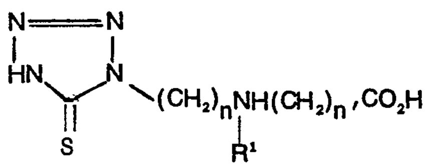

(I)

ses sels de métal alcalin et d'ammonium, dans laquelle n varie de deux à quatre inclusivement, n' varie de un à quatre inclusivement et $R^1$ représente un atome d'hydrogène ou un groupe alcoyle en $C_1$—$C_4$.

2. Composé tel que revendiqué dans la revendication 1, dans lequel $R^1$ représente un atome d'hydrogène.

3. Composé tel que revendiqué dans la revendication 1, dans lequel $R^1$ représente un groupe méthyle.

4. Composé tel que revendiqué dans l'une quelconque des revendications 1 à 3, dans lequel n est égal à 2 et n' est égal à 1.

5. Procédé de préparation d'un composé tel que revendiqué dans la revendication 1, caractérisé en ce que l'on fait réagir un dérivé d'aminoalcoyltétrazolyl thione de formule (II):

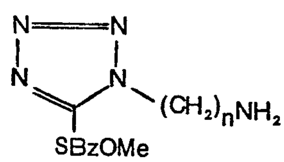

(II)

dans laquelle n est tel que défini dans la revendication 1 et BzOMe représente un groupe p-méthoxybenzyle, avec un bromoester de formule (III):

$$Br(CH_2)_{n'}CO_2C_2H_5$$

(III)

on fait réagir ensuite, éventuellement, l'ester du composé de formule (I) ainsi obtenu avec un agent alcoylant qui introduit un groupe alcoyle inférieur, on élimine ensuite le groupe p-méthoxybenzyle, on hydrolyse le groupe ester et on transforme éventuellement l'acide libre en un sel.

**Patentansprüche**

1. Eine Verbindung der Formel I

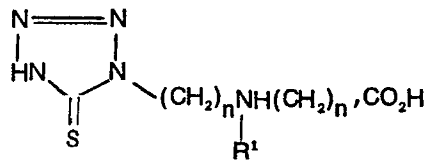

(I)

und ihre Alkalimetall- und Ammoniumsalze, wobei n einen Wert von 2 bis 4 einschließlich und n' von 1 bis 4 einschließlich aufweist und $R^1$ ein Wasserstoffatom oder einen $C_{1-4}$-Alkylrest bedeutet.

2. Verbindung nach Anspruch 1, in der $R^1$ ein Wasserstoffatom bedeutet.

3. Verbindung nach Anspruch 1, in der $R^1$ eine Methylgruppe bedeutet.

4. Verbindung nach Anspruch 1 bis 3, in der n den Wert 2 und n' den Wert 1 haben.

5. Verfahren zur Herstellung einer Verbindung nach Anspruch 1, dadurch gekennzeichnet, daß man ein Aminoalkyltetrazolylthion-Derivat der Formel II

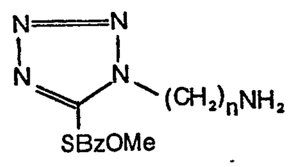

(II)

in der n die in Anspruch 1 angegebene Bedeutung hat und BzOMe eine p-Methoxybenzylgruppe darstellt, mit einem Bromester der Formel III umsetzt:

$$Br(CH_2)_{n'}CO_2C_2H_5$$

(III)

und gegebenenfalls den erhaltenen Ester der Verbindung der Formel I mit einem Alkylierrungsmittel umsetzt, welches einen niederen Alkylrest einführt, danach die p-Methoxybenzylgruppe abspaltet, die Estergruppe hydrolysiert und gegebenenfalls die freie Säure in ein Salz umwandelt.